# EUROPÄISCHE PATENTSCHRIFT

(11) **EP 1 928 426 B1**
(45) Veröffentlichungstag und Bekanntmachung des Hinweises auf die Patenterteilung: **01.10.2014**
(21) Anmeldenummer: 06777115.4
(22) Anmeldetag: 30.08.2006
(51) Int. Cl.: A61K 9/20, A61K 9/48, A61K 47/26, A61K 35/74, B65D 81/26

(54) **VERFAHREN ZUR STABILISIERUNG VON PHARMAZEUTISCHEN DARREICHUNGSFORMEN ENTHALTEND MIKROORGANISMEN**
METHOD FOR STABILISING PHARMACEUTICAL ADMINISTRATION FORMS THAT CONTAIN MICRO-ORGANISMS
PROCEDE POUR STABILISER DES FORMES GALENIQUES PHARMACEUTIQUES CONTENANT DES MICRO-ORGANISMES

(30) Priorität: 29.09.2005 EP 05021254
(43) Veröffentlichungstag der Anmeldung: 11.06.2008
(73) Patentinhaber: Merck Patent GmbH, 64293 Darmstadt (DE)
(72) Erfinder: RUDOLPH, Markus, 1267 Vich (CH); HENKE, Stefan, 57548 Kirchen (DE); MANNECK, Iris, 1184 Luins (DE); PEITZ, Holger, 65462 Ginsheim (DE); CHRIST, Andrea, 69115 Heidelberg (DE)
(86) Internationale Anmeldenummer: PCT/EP2006/008468
(87) Internationale Veröffentlichungsnummer: WO 2007/036278

(56) Entgegenhaltungen:
- EP-A- 1 072 258
- WO-A-98/41103
- WO-A-2005/063200
- US-A- 4 667 814
- ANONYMUS: "Desiccant film packages nearing market" IN-PHARMA TECHNOLOGIST.COM, [Online] 24. August 2005 (2005-08-24), XP002437467 Gefunden im Internet: URL:http://www.in-pharmatechnologist.com/n ews-by-product/news.asp?id=62058&idCat=66& k=alcan--desiccant-packaging> [gefunden am 2007-06-11]

## Beschreibung

Die Erfindung betrifft ein Verfahren zur Stabilisierung von festen pharmazeutischen Darreichungsformen, die wenigstens eine Gattung von Mikroorganismen enthält, sowie eine Packung umfassend ein Packmittel sowie eine feste pharmazeutische Darreichungsfom, die wenigstens eine Gattung von Mikroorganismen enthält.

Mikroorganismen sind vielfach in festen pharmazeutischen Darreichungsformen enthalten, wobei diese oral, vaginal oder anal verabreicht werden können. So werden beispielsweise probiotische Mikroorganismen in oral zu verabreichenden Arzneimitteln eingesetzt, um Symptome, die eine gestörte oder geschädigte Darmflora hervorruft, zu lindern oder zu beseitigen. Ein weiteres Beispiel sind Mikroorganismenkulturen enthaltende Vaginalsuppositorien, beispielsweise des Stammes Lactobacillus acidophilus, die zur Stabilisierung der Vaginalflora eingesetzt werden und zu einer Verminderung der Rezidivhäufigkeit von Harnwegsinfektionen führen (Reid G. et al.: Influence of three day antimicrobial therapy of lactobacillus vaginal suppositories on recurrence of uninary tract infections, Clin Ther 1992; 14: 11-6).

Zur Wirksamkeit der Präparate ist es erforderlich, dass die in diesen enthaltenen Mikroorganismen zum Zeitpunkt der Applikation in lebender Form vorliegen und dass sie in der Lage sind sich zu vermehren. Ziel ist es daher bei der Herstellung von Mikroorganismen enthaltenden pharmazeutischen Darreichungsformen und bei deren anschließenden Lagerung die Aktivität der enthaltenen Mikroorganismen soweit wie möglich zu erhalten.

Aus dem Stand der Technik sind Verfahren zur Herstellung und Lagerung von Mikroorganismen enthaltenden Arzneimitteln bekannt. So beschreibt beispielsweise EP 0 131 114 A1 ein Lactobacillus-Präparat durch Auftragen einer Suspension der Bakterien auf eine pulver- oder granulatförmige Trägermasse und deren anschließende Trocknung. Nach Herstellung wird das Präparat in ein Packmittel mit sauerstofffreier Schutzgasatmosphäre verbracht, um die Aktivität der enthaltenen Mikroorganismen während seiner Lagerung zu erhalten.

DE 198 19 475 beschreibt ein Trocknungsverfahren für Mikroorganismenkulturen, das deren Langzeit-Lagerstabilität erhöhen soll. Als Zielgröße wird eine Wasseraktivität (a_{w}) im Trocknungsgut von weniger als 0,15 angestrebt. Um diese Zielgröße zu erreichen, muss das Trocknungsgut im Wirbelschichtverfahren getrocknet werden und/oder es müssen Hilfsstoffe zugesetzt werden, die einen a_{w}-Wert von ≤ 0,01 aufweisen.

WO 2005063200 A2 beschreibt eine probiotische Tablette, die in einem Behälter aufbewahrt wird, der ein feuchtabsorbierendes Mittel zur Absorption von Wasser enthält, so dass die Wasseraktivität in dem Bereich, der die Tablette umgibt, herabgesetzt wird.

Die aus dem Stand der Technik bekannten Verfahren zur Erhöhung der Lagestabilität sind wenigstens aus einem der folgenden Gründe nicht zufriedenstellend:
1. die Verfahren sind technisch sehr aufwändig;
2. die Anforderung an die Wasseraktivität der Hilfsstoffe ist zu hoch;
3. die Lagerstabilität der Darreichungsformen ist zu gering;
4. die Verfahren sind kommerziell zu teuer;
5. die Verfahren bewirken eine thermische Schädigung der Mikroorganismenkulturen und/oder der Hilfs- und Wirkstoffe.

Es war Aufgabe der vorliegenden Erfindung ein Verfahren bereitzustellen, das die beschriebenen Nachteile des Standes der Technik nicht mehr aufweist. Das Verfahren sollte insbesondere einfach und preiswert durchzuführen sein, den Einsatz von Hilfsstoffen mit jeglicher Wasseraktivität erlauben, zu keiner thermischen Belastung der Mikroorganismen sowie der diese enthaltenden Darreichungsform führen und bewirken, dass die Aktivität der Mikroorganismen über die gesamte Lagerungszeit des Arzneimittels, d. h. bis zu dessen Einnahme durch den Patienten, weitgehend erhalten bleibt.

Die Aufgabe konnte überraschenderweise gelöst werden, indem eine feste, Mikroorganismen enthaltende Darreichungsform, die gemäß dem Stand der Technik hergestellt wurde, in ein Packmittel eingebracht wurde, in dessen Innenwandung/en zumindest teilflächig ein Absorptionsmittel und mindestens ein Kanalbildner eingebettet ist. Gegenstand der vorliegenden Erfindung ist daher ein Verfahren zur Stabilisierung einer festen pharmazeutischen Darreichungsform enthaltend mindestens eine Mikroorganismenkultur, das dadurch gekennzeichnet ist, dass die feste pharmazeutische Darreichungsform in ein Packmittel aus Polymer eingebracht wird, in dessen Innenwandung/en zumindest teilflächig ein Absorptionsmittel und mindestens ein Kanalbildner eingebettet ist, wobei als kanalbildner Polyglykole, Ethylvinylalkohole, Glycerin, Polyvinylalkohole, Polyvinylpyrrolidon, Vinylpyrrolidon, N-Methylpyrrolidon, Polysaccharide, Saccharide und/oder Zuckeralkohole und als Trockenmittel ein Molekularsieb oder kieselgel enthalten ist. Nach Einbringung der festen pharmazeutischen Darreichungsform in das Packmittel wird dieses, beispielsweise mit einem Deckel, verschlossen. Unter Innenwandung/en wird die nach innen gerichtete Fläche der Wand/Wände des Packmittels verstanden, also die Fläche/n des Packmittels, die im Kontakt zu der hierin enthaltenen festen pharmazeutischen Darreichungsform steht/stehen.

Das Verfahren kann für alle festen pharmazeutischen Darreichungsformen verwendet werden, die bei Raumtemperatur im festen Aggregatzustand vorliegen und z. B. zur oralen, analen oder vaginalen Verabreichung vorgesehen sind. Umfasst sind alle festen pharmazeutischen Darreichungsformen, die nach Entnahme aus dem Packmittel zur direkten Verabreichung vorgesehen sind, wie z. B. Tabletten, Dragees, Hartkapseln, Granulate, Pellets, Pulver, Pellets, Suppositorien, aber auch solche, die vor Verabreichung noch in die verabreichungsfähige Form überführt werden müssen, wie z. B. Trockensäfte, beispielsweise in Form von Pulvern, die vor Verabreichung noch in Lösung überführt werden müssen. Vorzugsweise ist die pharmazeutische Darreichungsform eine Tablette, ein Dragee, eine Hartkapsel, ein Granulat, ein Suppositorium, ein Pellet oder ein Pulver. Hartkapseln besitzen Hüllen ohne Weichmacherzusätze, sind in Ober- und Unterteil teilbar und bestehen beispielsweise aus Gelatine oder Stärke.

Pharmazeutische Darreichungsform wird vor- und nachstehend als Bezeichnung von verschiedenen technischen Darreichungsformen verstanden, wie sie für die Verabreichung von Arzneistoffen an Menschen oder Tieren bekannt sind. Der Ausdruck pharmazeutische Darreichungsform ist somit unabhängig von einem bestimmten rechtlichen Status und keineswegs auf Arzneimittel beschränkt, als Inhaltsstoffe können verschiedene Stoffe wie z. B. Arzneistoffe, Nahrungsergänzungsstoffe und/oder Functional Ingredients enthalten sein. Beispiele für pharmazeutische Darreichungsformen im Sinne der vorliegenden Erfindung können als Arzneimittel und Nahrungsergänzungsmittel vorliegen.

Als Mikroorganismen können alle Mikroorganismen enthalten sein, die im gesunden menschlichen oder tierischen Körper entweder selbst üblicherweise vorkommen oder die eine gesundheitsfördernde Wirkung auf den gesunden, gestörten oder erkrankten menschlichen oder tierischen Körper haben. Mikroorganismen, die enthalten sein können, sind z. B. Bakterien, Pilze und/oder Hefen.

Bevorzugte Mikroorganismen sind lebende Hefen, wie z. B. Saccharomyces boulardii, und/oder Bakterien, besonders bevorzugt Bakterien, ganz besonders bevorzugt probiotische Bakterien, wie z. B. Lactobacillen, Bifidobakterien und/oder Streptokokken. Besonders bevorzugt sind dabei die Spezies Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobakterium longum, Bifidobakterium bifidum, Bifidobakterium longum, Bifidobacterium lactis, Bifidobacterium brevis, Bifidobacterium animalis, Bifidobacterium adolescentis, Bifidobacterium infantis, Streptococcus thermophilus und/oder Lactococcus lactis.

Packmittel sind Behältnisse aus Polymeren. Absorptionsmittel und Kanalbildner können gemeinsam entweder direkt in der/den Innenwandung/en des das Behältnis ausbildenden Polymeren enthalten oder als Schicht auf der/den Innenwandung/en des Behältnisses aus Polymeren aufgebracht sein. Ebenso können Absorptionsmittel und Kanalbildner in ein Inlay eingebettet werden, das als Einschub in das Packmittel eingebracht wird, sodass zumindest ein Teil der Innenwandungen des Packmittels hierdurch ausgekleidet werden.

Behältnisse im Sinne der vorliegenden Erfindung sind sowohl Einzeldosenbehältnisse wie z. B. Durchdrückpackungen (Blisterpackungen) als auch Mehrdosenbehältnisse wie z. B. Schraubdeckelbehältnisse oder Tablettenröhrchen.

Polymere, die in Mischung mit Absorptionsmittel und Kanalbildner eingesetzt werden können, sind insbesondere Thermoplaste wie z.B. Polyolefine, wie Polyethylen und/oder Polypropylene, Polyisoprene, Polybutadiene, Polybutene, Polysiloxane, Polyamide, Ethylen-Vinylacetat-Copolymere, Ethylen-Methacrylat-Copolymere, Polystyrole, Polyester, Polyanhydride, Polyacrylatnitrile, Polysulfonate, Polyesteramide, Polyacrylatester, Propylen-Maleinsäureanhydrid, Polyethylen- Maleinsäureanhydrid, Polyethylen-Urethane, Polyethylen-Ethylvinylalkohole, Polyethylen-Nylon und/oder Polyurethane sein. Die an ihrer Innenfläche mit Absorptionsmittel und Kanalbildner ausgerüsteten Wände weisen, bezogen auf das Gesamtgewicht der Mischung aus Polymer, Kanalbildner und Absorptionsstoffen, einen Gehalt an Polymer von 10 - 90 Gew.-% auf.

Als Absorptionsmittel kann grundsätzlich jede Art von Trockenmitteln, d. h. feuchtigkeitsbindende Bindemittel, enthalten sein. Es kommen drei Gruppen von Trockenmitteln in Betracht:
Die erste Gruppe beinhaltet chemische Stoffe, die mit Wasser Hydrate bilden. Beispiele derartige chemische Stoffe sind wasserfreie Salze, die dazu neigen Wasser oder Feuchtigkeit zu absorbieren, und hierbei ein stabiles Hydrat bilden. Es wird die Feuchtigkeit gebunden und deren Freisetzung durch eine chemische Reaktion verhindert.

Die zweite Gruppe der Trockenmittel enthält Stoffe, die reaktiv sind. Die Stoffe reagieren mit Wasser oder Feuchtigkeit, indem sie einen neuen Stoff bilden. Die neu gebildeten Stoffe sind normalerweise bei niedrigen Temperaturen stabil, die nur unter Aufwendung hoher Energie reversibel ist. Diese Art von Trockenmitteln wird hauptsächlich zum Trocknen von Lösungsmitteln und als Wasser absorbierendes Material bei Polymeren, die selber in einem feuchtigkeitsreduzierten Zustand bleiben müssen, verwendet.

Die Gruppe der Trockenmittel bindet die Feuchtigkeit durch physikalisch Adsorption. Das Trockenmittel enthält Teilchen mit feinen Kapillaren, in welche die Feuchtigkeit eingezogen wird. Dabei bestimmen die Porengröße der Kapillaren sowie deren Dichte im Trocknungsmittel die Absorptionseigenschaften. Trockenmittel sind Molekularsiebe oder Kieselgele. Trockenmittel sind im Packmittel enthalten, da sie weitgehend inert und wasserunlöslich sind. Besonders bevorzugt sind dabei Molekularsiebe mit einer Porengröße von 3 bis 15 Angström und/oder Kieselgele mit einer Porengröße von 24 Angström.

Die Kanalbildner sind Polyglykole. Ethylvinylalkole, Glycerin, Polyvinylalkohole, Polyvinylpyrrolidon, Vinylpyrrolidon, N-Methylpyrrolidon, Polysaccharide, Saccharide und/oder Zuckeralkohole. Als Polyglykole werden Polyethylenglykol und/oder Polypropylenglykol bevorzugt. Als Saccharide können z.B. Glucose, Mannose, Galactose und/oder Fructose verwendet werden. Als Zuckeralkohole in Frage kommen z.B. Mannitol, Sorbitol, Hexitol, Dulcitol, Xylitol, Ribitol und/oder Erythrol. Unter Polysacchariden zu verstehen sind z.B. Dextrine und/oder hydrolisierte Stärke.

In den mit Absorptionsmitteln und Kanalbildnern ausgerüsteten Innenwandungen können die Kanalbildner, bezogen auf das Gesamtgewicht der Mischung aus Polymer, Kanalbildner und Absorptionsmittel, einen Anteil von 10 - 40 Gew.-% aufweisen.

Absorptionsmittel und Kanalbildner sind in der/den Innenwandung/en des Behältnisses teilflächig oder ganzflächig eingebettet. Teilflächig bedeutet, dass zumindest ein Teil der gesamten die Innenwandung/en ausbildenden Fläche des Behältnisses Absorptionsmittel und Kanalbildner enthält. Ganzflächig bedeutet, dass die gesamten, die Innenwandungen ausbildende Fläche des Behältnisses Absorptionsmittel und Kanalbildner enthält. Nach einer vorteilhaften Ausführungsform sind, bezogen auf die gesamte Innenfläche des Behältnisses, Absorptionsmittel und Kanalbildner in mindestens 10 %, bevorzugt in mindestens 50 %, besonders bevorzugt in mindestens 90 % der Innenwandungen enthalten.

Polymere, die Absorptionsmittel und Kanalbildner enthalten, und Behältnisse aus diesen, die als Packmittel für das erfindungsgemäße Verfahren verwendet werden können, sind dem Stand der Technik bekannt und werden z. B. beschrieben in WO 97/32663 A1, EP 1000873 A2 und WO 03/086900 A1, EP 1421991 A1, WO 00/76879 A1. Packmittel, die für das Verfahren der vorliegenden Erfindung eingesetzt werden können, sind kommerziell erhältlich und werden beispielsweise von der Firma Capitol Specialty Plastics Inc., 2039 McMillan Street Auburn, Alabama, USA, unter dem Warenzeichen Activ-Vial oder von der Firma Süd Chemie, Ostenrieder Str. 15, 85368 Moosburg, Deutschland, unter dem Markennamen 2 AP Multipolymer angeboten.

Das erfindungsgemäße Verfahren ermöglicht die preiswerte Bereitstellung von stabilen, mindestens eine Mikroorganismenkultur enthaltende feste pharmazeutische Darreichungsform. Diese können beispielsweise mit preiswerten Rohstoffen hergestellt werden, die gemäß dem Stand der Technik auf Grund ihrer hohen Wasseraktivitätswerte nicht als Ausgangsmaterial verwendet werden können oder in zusätzlichen Schritten vor und/oder nach Verarbeitung zur pharmazeutischen Darreichungsform getrocknet werden müssen.

Überraschenderweise ermöglicht das erfindungsgemäße Verfahren auch die Bereitstellung von vermarktungsfähigen Produkten aus mindestens eine Mikroorganismenkultur enthaltenden festen pharmazeutischen Darreichungsformen, die bisher gemäß dem Stand der Technik für die Vermarktung ungeeignet, da sie nicht ausreichend lagerstabil sind. Nach Überführung der Darreichungsform in das Packmittel wird der Darreichungsform durch das in der/den Innenwandung/en des Packmittels enthaltene Absorptionsmittel kontinuierlich und über einen langen Zeitraum Wasser entzogen. Der Wasserentzug erfolgt großflächig und unter milden Bedingungen und führt so zur Stabilisierung der festen pharmazeutischen Darreichungsform während seiner Lagerung.

Eine mit dem erfindungsgemäßen Verfahren vergleichbare Stabilisierung einer mindestens eine Mikroorganismenkultur enthaltenden festen pharmazeutischen Darreichungsform ist auch mit einer der Herstellung nachgelagerten Trocknung nicht ohne weiteres zu erreichen, da eine lang andauernde Trocknung bei vergleichbar milden Bedingungen aus Zeit- und Kostengründen praktisch nicht durchführbar ist und eine Trocknung bei erhöhter Temperatur zur Schädigung der pharmazeutischen Darreichungsform führt, insbesondere von den hierin enthaltenen Mikroorganismenkulturen.

Die stabilisierende Wirkung des erfindungsgemäßen Verfahrens beruht auf dem Einfluss des Packmittels auf die feste pharmazeutische Darreichungsform, die hierdurch lagerstabil zur Verfügung gestellt werden kann. Die Erzielung der erfindungsgemäßen Wirkung erfordert somit, dass die feste pharmazeutische Darreichungsform in dem Packmittel enthalten ist, Darreichungsform und Packmittel also zusammen als Packung vorliegen.

Gegenstand der Erfindung ist daher auch eine Packung umfassend ein Packmittel nach Anspruch 5.

Die Packung kann jede mindestens eine Mikroorganismenkultur enthaltende feste pharmazeutische Darreichungsform enthalten. Nach einer bevorzugten Ausführungsform der Erfindung enthält die Packung als feste pharmazeutische Darreichungsform eine zur oralen Verabreichung bestimmte feste pharmazeutische Darreichungsform, insbesondere eine Tablette, ein Dragee, eine Hartkapsel, ein Granulat, ein Suppositorium oder ein Pulver, Die in der Packung enthaltene pharmazeutische Darreichungsform kann Mikroorganismen in jeder Anzahl enthalten, die für den jeweiligen Zweck erforderlich ist. Vorzugsweise enthält die in der Packung enthaltene feste pharmazeutische Darreichungsform 10³ bis 10¹², besonders bevorzugt 10⁵ bis 10¹¹ und ganz besonders bevorzugt 10⁷ bis 10¹⁰ probiotische Mikroorganismen.

Nach peroraler Verabreichung werden probiotische Mikroorganismen bei der vorgeschalteten Magenpassage zum großen Teil inaktiviert bevor diese im menschlichen oder tierischen Darm ihre gesundheitsfördernde Wirkung erzielen können. Zur Sicherstellung einer ausreichend hohen Aktivität der probiotischen Mikroorganismen im menschlichen und tierischen Darm ist es daher bevorzugt, dass die in der Packung enthaltene feste orale pharmazeutische Darreichungsform mit einem Überzug versehen ist, der die Mikroorganismen vor deren Inaktivierung während der Magenpassage schützt, sich danach aber im Darm auflöst, sodass die Mikroorganismen hier freigesetzt werden. In Frage kommen alle Überzüge, bevorzugt pHgesteuerte Systeme wie z. B. magensaftresistente Überzüge, d. h.. Überzüge aus Materialien, die im sauren Magensaft unlöslich und im (alkalischeren) Darm löslich sind, oder zeitgesteuerte Überzüge, d. h. Überzüge, die sich nach oraler Verabreichung an den Mensch oder das Tier unabhängig vom pH-Wert der jeweiligen Umgebung nach einem vorbestimmten Zeitraum auflösen, wobei dieser so eingestellt ist, dass er der Passagedauer der Darreichungsform ab Einnahme des Präparats bis zu dessen Ankunft am Zielort Darm entspricht. Alternativ können anstatt der gesamten festen pharmazeutischen Darreichungsform auch die Mikroorganismen selbst mit dem schützenden Überzug versehen sein.

Nach einer weiterhin bevorzugten Ausführungsform enthält die in der Packung enthaltene feste pharmazeutische Darreichungsform, insbesondere wenn sie zur oralen Verabreichung vorgesehen ist, zusätzlich zu den probiotischen Mikroorganismen weitere ernährungsrelevante Zusätze. Ernährungsrelevante Zusätze, die enthalten sein können, sind vorzugsweise Vitamine, Mineralstoffe, Spurenelemente, Ballaststoffe, Enzyme, Pflanzenextrakte, Eiweiße, Kohlenhydrate und/oder Fette. Enthält die orale Darreichungsform ernährungsrelevante Zusätze, deren Verdauung bereits im Magen beginnt, wie z.B. Eiweiße, so ist es wichtig, dass diese ernährungsrelevanten Zusätze zumindest nicht vollständig von dem Überzug umschlossen sind.

Orale pharmazeutische Darreichungsformen, die probiotische Mikroorganismen und weitere, ernährungsrelevante Zusätze enthalten, sind bekannt aus EP 931 543 A1. Wie dort beschrieben sind Formulierungen, die probiotische Mikroorganismen zusammen mit den beschriebenen ernährungsrelevanten Zusätzen enthalten, instabil. Um dennoch eine probiotische Mikroorganismen und ernährungsrelevante Zusätze enthaltende orale Darreichungsform zur Verfügung zu stellen, wird vorgeschlagen, die probiotische Mikroorganismen und die ernährungsrelevanten Zusätze auf voneinander getrennte Schichten einer mehrschichtigen Tablette zu verteilen. Zum Erreichen einer guten Stabilität soll die Mikroorganismen enthaltende.Schicht einen sehr niedrigen Wassergehalt aufweisen, was dadurch erreicht wird, dass die Mikroorganismenkulturen und das Trägermaterial vor deren Vermischung sehr sorgfältig getrocknet werden. Die erforderliche Trocknung von Mikroorganismen und Trägermaterial sowie die Formulierung als Mehrschichttablette ist jedoch apparativ und zeitlich aufwändig und führt insgesamt zu hohen Herstellkosten.

Überraschenderweise wurde gefunden, dass feste pharmazeutische Darreichungsformen enthaltend mindestens eine Mikroorganismenkultur und ernährungsrelevante Zusätze lagerstabil bereitgestellt werden können, ohne dass Mikroorganismen und Trägermaterial vorgetrocknet werden und/oder dass die Mikroorganismen und die ernährungsrelevanten Zusätze in jeweils voneinander getrennten Schichten enthalten sind, indem die die Mikroorganismen und ernährungsrelevante Zusätze enthaltende feste pharmazeutische Darreichungsform in ein Packmittel eingebracht wird, in dessen Innenwandung/en zumindest teilflächig mindestens ein Kanalbildner zusammen mit mindestens einem Absorptionsmittel eingebettet ist, sodass eine Packung umfassend besagtes Packmittel sowie die feste, Mikroorganismen enthaltende pharmazeutische Darreichungsform vorliegt. Gegenstand der Erfindung ist daher auch eine Packung umfassend ein Packmittel, in dessen Innenwandung/en zumindest teilflächig mindestens ein Kanalbildner zusammen mit mindestens einem Absorptionsmittel eingebettet ist, sowie eine feste pharmazeutische Darreichurigsform enthaltend mindestens eine Mikroorganismenkultur sowie mindestens einen ernährungsrelevanten Zusatz. Vorteilhaft können der/die ernährungsrelevante/n Zusatz/Zusätze und die Mikroorganismen in der in der Packung enthaltenen pharmazeutischen Darreichungsform in Mischung zueinander vorliegen, ohne dass dies zu einem instabilen Produkt führt. Die in dem Stand der Technik beschriebene Beschränkung auf Formulierungen mit schichtartigem Aufbau, insbesondere auf eine Mehrschichttablette, entfällt.

Vitamine, die in der in der erfindungsgemäßen Packung enthaltenen festen pharmazeutischen Darreichungsform bevorzugt enthalten sind, sind Vitamin A (β-Carotin), Carotinoide, Vitamin D, Vitamin C, Vitamin E, Vitamine des B-Komplexes und/oder Vitamin K sowie verwandte Verbindungen hiervon mit vergleichbarer Aktivität. Als Vitamine des B- Komplexes bevorzugt enthalten sein kann Folsäure, Tetrahydrofolsäure und/oder dessen Derivate, insbesondere (6S)-Tetrahydrofolsäure, 5-Methyl-(6S)-Tetrahydrofolsäure, 5-Formyl-(6S)-Tetrahydrofolsäure, 10-Formyl-(6R)-Tetrahydrofolsäure, 5,10-Methylen-(6R)-Tetrahydrofolsäure, 5,10-Methenyl-(6R)-Tetrahydrofolsäure und/oder 5-Formimino-(6S)-Tetrahydrofolsäure. Die Menge an den Vitaminen richtet sich in der Regel nach der empfohlenen Mindestbedarfdosis für das jeweilige Vitamin, wobei diese jedoch auch um durchschnittlich 50 bis 300 % überschritten werden kann. Bevorzugte Bereiche sind für das Vitamin C zwischen 50 und 300 mg, für das Vitamin E 10 bis 50 mg, für das Vitamin A ≤ 1,5 mg und für die Vitamine des B- Komplexes 10 µg bis 20 mg.

Mineralstoffe, die in der in der erfindungsgemäßen Packung enthaltenen festen pharmazeutischen Darreichungsform bevorzugt enthalten sind, sind für den Verzehr geeignete, anorganische oder organische Natrium-, Kalium-, Calcium-, Magnesium-, Zink- und/oder Eisensalze, welche vorzugsweise als Carbonate, Bicarbonate, Phosphate, Biphosphate, Sulfate, Bisulfate, Chloride, Fluoride, Citrate und/oder Lactate vorliegen. Der Anteil an Mineralstoffen bezogen auf das Gesamtgewicht der festen pharmazeutischen Darreichungsform beträgt vorzugsweise von 20 bis 40 Gew.-%. Vorzugsweise enthält die feste Darreichungsform als Spurenelemente Silicium, Chrom, Mangan, Jod, Molybdän und/oder Selen.

Als Ballaststoffe enthält die in der erfindungsgemäßen Packung enthaltene feste pharmazeutische Darreichungsform vorzugsweise Sojakleie, Maiskleie, Weizenkleie und/oder Getreideschrot, besonders bevorzugt Sojakleie. Der Anteil an Ballaststoffen, bezogen auf das Gesamtgewicht der festen pharmazeutischen Darreichungsform, beträgt vorzugsweise 2 bis 50 Gew.-%.

Bevorzugte Enzyme bzw. Co-Enzyme sind Lipasen und/oder Proteasen, bzw. CoEnzym Q, Superoxiddismutase und/oder Gluthathionperoxidase, die die Magen und/oder Darmfunktion und/oder den Stoffwechsel fördern. Diese können in an sich bekannter Menge und in an sich bekannter Form eingebracht werden.

Die in der erfindungsgemäßen Packung enthaltene feste pharmazeutische Darreichungsform kann außerdem weitere prebiotische Substanzen, vorzugsweise Oligofructose und/oder andere Oligozucker enthalten.

Als Pflanzenextrakte bevorzugt sind Trockenextrakte und hier insbesondere solche, die Bioflavonoide, Polyphenole, Phytoöstrogene und/oder Saponine enthalten, wie z.B. aus Echinaceae.

Vorzugsweise enthält die in der erfindungsgemäßen Packung enthaltene feste pharmazeutische Darreichungsform als Eiweiße Sojaprotein und/oder Molkenprotein und/oder als Fette solche Fette, die mehrfach ungesättigte Fettsäuren enthalten.

Die in der erfindungsgemäßen Packung enthaltene feste pharmazeutische Darreichungsform kann außerdem je nach Ausführungsform übliche Hilfs-und Zusatzstoffe enthalten. Die Auswahl der Hilfs- und/oder Zusatzstoffe hängt auch von den lebensmittelrechtlichen Bestimmungen des Landes ab, in dem die in der Packung enthaltene feste pharmazeutische Darreichungsform zum Einsatz kommen soll. Als Hilfs- und/oder Zusatzstoffe kommen, beispielsweise für Tabletten, Mehrschichttabletten, Dragees, Hartkapseln, Granulate, Pelletzubereitungen und/oder Pulver, Stärke (z.B. Maisstärke), Talkum, mikrokristalline Cellulose, Lactose, hochdisperses Siliciumdioxid, Polyvinylpyrrolidon und/oder Cellulosepulver zum Einsatz. Als weitere Bestandteile können als Bindemittel und/oder Trennmittel Kohlenhydrate, wie beispielsweise Mannit, Sorbit, Xylit, Glucose, Sucrose, Fructose, Maltose, Dextrose, Maltodextrin und/oder Kaolin und/oder Cellulosederivate, wie beispielsweise Methylcellulose, Hydroxypropylcellulose und/oder Hydroxypropylmethylcellulose und/oder Calciumcarbonat, Calcium-, Magnesium- und/oder Glycerinstearat enthalten sein. Des weiteren kann die in der Packung enthaltene feste pharmazeutische Darreichungsform auch Farb-, Geschmacks- und/oder Aromastoffe, sowie Gleitmittel, Antioxidantien und/oder Stabilisatoren enthalten. Der Gehalt dieser Grundlagenstoffe richtet sich einerseits nach dem angestrebten Gehalt an probiotischen Mikroorganismen, Vitaminen, Enzymen, Ballaststoffen u.s.w. und andererseits nach Kriterien, die die mechanisch-physikalischen Eigenschaften der oralen Darreichungsform bestimmen, wie z.B. Härte, Verpressbarkeit, Größe, Farbe und/oder Form.

Die Herstellung der erfindungsgemäßen oralen Darreichungsform kann nach verschiedenen dem Fachmann bekannten Methoden durchgeführt werden. Diese Methoden sind z.B. aus H. Sucker, P. Fuchs, P. Speiser, "Pharmazeutische Technologie", Stuttgart 1978 oder K.H. Bauer, K.H. Frömming, C. Führer, "Pharmazeutische Technologie", Stuttgart 1986 bekannt. Sie werden hiermit als Referenz eingeführt und sind somit Teil der Offenbarung.

Die Beispiele, ohne hierauf beschränkt zu sein, erläutern die Erfindung.

### Beispiel 1:

### 3-Schichttablette analog in EP 931 543 A1 enthaltend probiotische Bakterien

### Herstellung:

Mischungen aus 3 Gew.-% Bakterienzubereitung (enthaltend Lactobacillus gasseri, Bifidobacterium bifidum, Bifidobacterium longum), 10,5 Gew.-% Inulin, 8,6 Gew.-% Kalziumphosphat, 5,7 Gew.-% Cellulose, 2,3 Gew.-% Hilfsstoffe (Sprengmittel, Trennmittel) (1. Schicht), Mineralstoffe, Spurenelemente, Farbstoffe, Sprengmittel, Trennmittel, Cellulose (2. Schicht) sowie Vitamine, Spurenelemente, Sprengmittel, Trennmittel und Cellulose (3. Schicht) (Prozentangaben jeweils bezogen auf Gesamttablettengewicht), werden nacheinander auf einer 3-Schichttablettenpresse (Rundläufer) der Firma E. Hata zu einer oblongförmigen 3-Schichtentablette mit den Maßen 18 mm x 8 mm verpresst. Die erhaltenen Tabletten werden anschließend mit einem Filmüberzug versehen (aus wässriger Lösung enthaltend Hydroxypropylmethylcellulose, Hydroxypropylcellulose und ein Trennmittel), der Überzug betrug, bezogen auf das Gewicht des Kernes, 5 Gew.-%, entsprechend 11 mg/cm² Tablettenoberfläche. Es werden überzogene 3-Schichtentabletten erhalten mit einem Gewicht von jeweils 1050 mg.

### Lagerung und Prüfung:

Die Stabilität der Filmtabletten wird in Haltbarkeitsstudien überprüft. Hierzu werden Filmtabletten entweder in ein Packmittel, in dessen Innenwandung ein Kanalbildner zusammen mit einem Absorptionsmittel eingebettet ist (Packmittel A), oder in eine Polypropylen-Dose mit Schraubdeckel (Packmittel B) eingebracht und bei 40°C/75%rF eingelagert. Nach vorbestimmten Zeiten wird ausgelagert und die jeweils enthaltene Mikroorganismenzahl nach dem Kochschen Plattengussverfahren durch Auszählen ermittelt. Die Ergebnisse sind in Tabelle 1 zusammengestellt (Mittelwert aus drei Chargen)

**Tabelle 1**

| Testparameter | Start | Lagerbedingung | |
|---|---|---|---|
| | | 40 °C/75 % rF | |
| | | 13 Wochen | 26 Wochen |
| | | Packmittel A | |
| Probiotische Kulturen [KBE] | 1,4·10⁸ | 5,9·10⁷ | 4,6·10⁷ |

| | | Packmittel B | |
|---|---|---|---|
| Probiotische Kulturen [KBE] | 1.4·10⁸ | 2,5·10³ | <1,0·10³ |

### Beispiel 2

### Tablette enthaltend Bakterien, Vitamine und Mineralstoffe

### Herstellung:

Eine Mischung aus 50 Gew.-% Sorbit, 7,7 Gew.-% Vitaminmischung, 24 Gew.-% Mineralstoffe, 3 Gew.-% Bakterienzubereitung (enthaltend Lactobacillus gasseri, Bifidobacterium bifidum, Bifidobacterium longum), 6 Gew.-% Trennmittel, 4,5 Gew.-% Farbstoffe, 2,3 Gew.-% Säuerungsmittel, 2,2 Gew.-%% Aromastoffe und 0,06 Gew.-% Süßstoffe wird auf einer Exzenterpresse E1 der Firma Fette zu einer leicht gewölbten, runden Tablette mit einem Gewicht von 1000 mg und einem Durchmesser von 15 mm verpresst.

Die Einlagerung und Stabilitätsprüfung erfolgt analog Beispiel 1. Packmittel B ist in diesem Beispiel eine Polypropylen-Dose mit Schraubdeckel und einer Trockenmittelkapsel. Die Ergebnisse sind in Tabelle 2 zusammengestellt.

**Tabelle 2**

| Testparameter | Start | Lagerbedingung | |
|---|---|---|---|
| | | 40 °C/75 % rF | |
| | | 13 Wochen | 26 Wochen |
| | | Packmittel A | |
| Probiotische Kulturen [KBE] | 5,3·10⁷ | 3,9·10⁷ | 3,0·10⁷ |

| | | Packmittel B | |
|---|---|---|---|
| Probiotische Kulturen [KBE] | 5.3·10⁷ | 5,4·10⁶ | <1,0.10³ |

### Beispiel 3

### Granulat enthaltend eine Bakterienzubereitung

In dem Granulator GPCG 3 (Düse 0,5 mm) der Firma Glatt wurde eine Bakterienzubereitung mit einer wässrigen Gelatinelösung bei einer Zulufttemperatur von 65°C, einer Ablufttemperatur von ca. 45°C und einer Sprührate von 12 g/min granuliert.

### Beispiel 4

### Hartgelatinekapsel enthaltend eine Bakterienzubereitung

Eine Mischung aus 98 % Bakterienzubereitung, 1 % Ascorbinsäure und 1 % Inulin wurde in Hartgelatinekaseln (Größe 0) abgefüllt.

### Beispiel 5

### Hartgelatinekapsel enthaltend eine Bakterienzubereitung

Eine Mischung mindestens enthaltend eine Bakterienzubereitung (enthaltend Lactobacillus casei, Lactococcus lactis, Lactobacillus acidophilus, Bifidobacterium bifidum) wurde in Hartgelatinekaseln (Größe 0) abgefüllt.

Die Einlagerung und Stabilitätsprüfung erfolgt analog Beispiel 1. Packmittel B ist in diesem Beispiel ein organisches Polymer/Alu-Blister. Die Ergebnisse sind in Tabelle 3 zusammengestellt.

**Tabelle 3**

| Testparameter | Start | Lagerbedingung | |
|---|---|---|---|
| | | 40 °C/75 % rF | |
| | | 13 Wochen | 26 Wochen |
| | | Packmittel A | |
| Probiotische Kulturen [KBE] | 1,1·10¹⁰ | 2.8·10⁹ | 1,6·10⁹ |

| | | Packmittel B | |
|---|---|---|---|
| Probiotische Kulturen [KBE] | 1.1·10¹⁰ | - | <1,0·10³ |

### Beispiel 6

### Hartgelatinekapsel enthaltend eine Bakterienzubereitung

Eine Mischung aus einer Bakterienzubereitung (enthaltend Lactobacillus GG), mikrokristalline Cellulose und Magnesiumstearat wurde in Hartgelatinekaseln (Größe 0) abgefüllt.

Die Einlagerung und Stabilitätsprüfung erfolgt analog Beispiel 1. Die Ergebnisse sind in Tabelle 4 zusammengestellt.

**Tabelle 4**

| Testparameter | Start | Lagerbedingung | |
|---|---|---|---|
| | | 40 °C/75 % rF | |
| | | 13 Wochen | 26 Wochen |
| | | Packmittel A | |
| Probiotische Kulturen [KBE] | 2,4·10¹⁰ | 1,2·10⁸ | 8,7·10⁷ |

### Beispiel 7

### Pulver enthaltend eine Bakterienzubereitung

Das abgefüllte Pulver enthält eine Bakterienzubereitung (enthaltend Lactobacillus GG), Maltodextrin, Sorbit und Saccharose.

Die Einlagerung und Stabilitätsprüfung erfolgt analog Beispiel 1. Die Ergebnisse sind in Tabelle 5 zusammengestellt.

**Tabelle 5**

| Testparameter | Start | Lagerbedingung | |
|---|---|---|---|
| | | 40 °C/75 % rF | |
| | | 13 Wochen | 26 Wochen |
| | | Packmittel A | |
| Probiotische Kulturen [KBE] | 5,7·10⁹ | 4,3·10⁸ | 1,3·10⁹ |

## Patentansprüche

1. Verfahren zur Stabilisierung einer festen pharmazeutischen Darreichungsform enthaltend mindestens eine Mikroorganismenkultur, **dadurch gekennzeichnet, dass** die feste pharmazeutische Darreichungsform in ein Packmittel aus Polymer eingebracht wird, in dessen Innenwandung/en zumindest teilflächig mindestens ein Kanalbildner zusammen mit mindestens einem Trockenmittel eingebettet ist, wobei als Kanalbildner Polyglykole, Ethylvinylalkohole, Glycerin, Polyvinylalkohole, Polyvinylpyrrolidon, Vinylpyrrolidon, N-Methylpyrrolidon, Polysaccharide, Saccharide und/oder Zuckeralkohole und als Trockenmittel ein Molekularsieb oder Kieselgel enthalten ist.

2. Verfahren nach Anspruch 1, **dadurch gekennzeichnet, dass** die feste pharmazeutische Darreichungsform Tablette, ein Dragee, eine Kapsel, ein Granulat, ein Suppositorium, ein Pellet oder ein Pulver ist.

3. Verfahren nach Anspruch 1 und/oder 2, **dadurch gekennzeichnet, dass** als Mikroorganismenkultur probiotische Mikroorganismen enthalten sind.

4. Verfahren nach Anspruch 3, **dadurch gekennzeichnet, dass** die probiotischen Mikroorganismen Lactobacillen, Bifidobakterien, oder Streptokokken, vorzugsweise Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobakterium longum, Bifidobakterium bifidum, Bifidobakterium longum, Bifidobacterium lactis, Bifidobacterium brevis, Bifidobacterium animalis, Bifidobacterium adolescentis, Bifidobacterium infantis, Streptococcus thermophilus und/oder Lactococcus lactis sind.

5. Packung umfassend ein Packmittel aus Polymer, in dessen Innenwandung/en zumindest teilflächig mindestens ein Kanalbildner zusammen mit mindestens einem Trockenmittel eingebettet ist sowie eine mindestens eine Mikroorganismenkultur enthaltende feste pharmazeutische Darreichungsform, die eine Tablette, ein Dragee, eine Kapsel, ein Granulat, ein Suppositorium, ein Pellet oder ein Pulver sein kann, wobei als Kanalbildner Polyglykole, Ethylvinylalkohole, Glycerin, Polyvinylalkohole, Polyvinylpyrrolidon, Vinylpyrrolidon, N-Methylpyrrolidon, Polysaccharide, Saccharide und/oder Zuckeralkohole und als Trockenmittel ein Molekularsieb oder Kieselgel enthalten ist.

6. Packung nach Anspruch 5, **dadurch gekennzeichnet, dass** die im Packmittel enthaltene feste pharmazeutische Darreichungsform zur oralen Verabreichung vorgesehen ist.

7. Packung nach Anspruch 5 und/oder 6, **dadurch gekennzeichnet, dass** die im Packmittel enthaltene feste pharmazeutische Darreichungsform 10³ bis 10¹² vorzugsweise 10⁵ bis 10¹¹ und besonders bevorzugt 10⁷ bis 10¹⁰ Mikroorganismen enthält.

8. Packung nach einem oder mehreren der Ansprüche 5 bis 7, **dadurch gekennzeichnet, dass** die im Packmittel enthaltene pharmazeutische Darreichungsform weitere ernährungsrelevante Zusätze, vorzugsweise Vitamine, Mineralstoffe, Spurenelemente, Ballaststoffe, Enzyme, Pflanzenextrakte, Eiweiße, Kohlenhydrate und/oder Fette enthält.

## Claims

1. Method for the stabilisation of a solid pharmaceutical administration form comprising at least one microorganism culture, **characterised in that** the solid pharmaceutical administration form is introduced into polymer packaging in the inner wall(s) of which at least one channel former together with at least one desiccant is embedded over at least part of the area, where the channel formers present are polyglycols, ethylvinyl alcohols, glycerol, polyvinyl alcohols, polyvinylpyrrolidone, vinylpyrrolidone, N-methylpyrrolidone, polysaccharides, saccharides and/or sugar alcohols and the desiccant present is a molecular sieve or silica gel.

2. Method according to Claim 1, **characterised in that** the solid pharmaceutical administration form is a tablet, a dragee, a capsule, a granular product, a suppository, a pellet or a powder.

3. Method according to Claim 1 and/or 2, **characterised in that** the microorganism culture present is probiotic microorganisms.

4. Method according to Claim 3, **characterised in that** the probiotic microorganisms are lactobacilli, bifidobacteria, or streptococci, preferably Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobacterium longum, Bifidobacterium bifdum, Bifidobacterium longum, Bifidobacterium lactis, Bifidobacterium brevis, Bifidobacterium animalis, Bifidobacterium adolescentis, Bifidobacterium infantis, Streptococcus thermophilus and/or Lactococcus lactis.

5. Pack comprising polymer packaging in the inner wall(s) of which at least one channel former together with at least one desiccant is embedded over at least part of the area, and a solid pharmaceutical administration form comprising at least one microorganism culture, which can be a tablet, a dragee, a capsule, a granular product, a suppository, a pellet or a powder, where the channel formers present are polyglycols, ethylvinyl alcohols, glycerol, polyvinyl alcohols, polyvinylpyrrolidone, vinylpyrrolidone, N-methylpyrrolidone, polysaccharides, saccharides and/or sugar alcohols and the desiccant present is a molecular sieve or silica gel.

6. Pack according to Claim 5, **characterised in that** the solid pharmaceutical administration form present in the packaging is intended for oral administration.

7. Pack according to Claim 5 and/or 6, **characterised in that** the solid pharmaceutical administration form present in the packaging comprises 10³ to 10¹², preferably 10⁵ to 10¹¹ and particularly preferably 10⁷ to 10¹⁰ microorganisms.

8. Pack according to one or more of Claims 5 to 7, **characterised in that** the pharmaceutical administration form present in the packaging comprises further nutrition-relevant additives, preferably vitamins, mineral substances, trace elements, dietary fibre, enzymes, plant extracts, proteins, carbohydrates and/or fats.

## Revendications

1. Procédé pour la stabilisation d'une forme solide pour administration pharmaceutique comprenant au moins une culture de microorganismes, **caractérisé en ce que** la forme solide pour administration pharmaceutique est introduite à l'intérieur d'un packaging en polymère dans la/les paroi(s) interne(s) duquel au moins un moyen de formation de rainure en association avec au moins un agent dessiccatif est noyé sur au moins une partie de la surface, où les moyens de formation de rainure présents sont des polyglycols, des alcools éthylvinyliques, du glycérol, des alcools polyvinyliques, du polyvinylpyrrolidone, du vinylpyrrolidone, du N-méthylpyrrolidone, des polysaccharides, des saccharides et/ou des alcools de sucre et l'agent dessiccatif présent est un tamis moléculaire ou un gel de silice.

2. Procédé selon la revendication 1, **caractérisé en ce que** la forme solide pour administration pharmaceutique est un comprimé, une dragée, une gélule, un produit granulaire, un suppositoire, une pastille ou une poudre.

3. Procédé selon la revendication 1 et/ou 2, **caractérisé en ce que** la culture de microorganismes présente est constituée par des microorganismes probiotiques.

4. Procédé selon la revendication 3, **caractérisé en ce que** les microorganismes probiotiques sont des lactobacilles, des bifidobactéries ou des streptocoques, de façon préférable Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobacterium longum, Bifidobacterium bifidum, Bifidobacterium longum, Bifidobacterium lactis, Bifidobacterium brevis, Bifidobacterium animalis, Bifidobacterium adolescentis, Bifidobacterium infantis, Streptococcus thermophilus et/ou Lactococcus lactis.

5. Pack comprenant un packaging en polymère dans la/les paroi(s) interne(s) duquel au moins un moyen de formation de rainure en association avec au moins un agent dessiccatif est noyé sur au moins une partie de la surface, et au moins une forme solide pour administration pharmaceutique comprenant au moins une culture de microorganismes, laquelle peut être un comprimé, une dragée, une gélule, un produit granulaire, un suppositoire, une pastille ou une poudre, où les moyens de formation de rainure présents sont des polyglycols, des alcools éthylvinyliques, du glycérol, des alcools polyvinyliques, du polyvinylpyrrolidone, du vinylpyrrolidone, du N-méthylpyrrolidone, des polysaccharides, des saccharides et/ou des alcools de sucre et l'agent dessiccatif présent est un tamis moléculaire ou un gel de silice.

6. Pack selon la revendication 5, **caractérisé en ce que** la forme solide pour administration pharmaceutique présente dans le packaging est destinée à une administration par voie orale.

7. Pack selon la revendication 5 et/ou 6, **caractérisé en ce que** la forme solide pour administration pharmaceutique présente dans le packaging comprend 10³ à 10¹², de façon préférable 10⁵ à 10¹¹ et de façon particulièrement préférable 10⁷ à 10¹⁰ microorganismes.

8. Pack selon une ou plusieurs des revendications 5 à 7, **caractérisé en ce que** la forme solide pour administration pharmaceutique présente dans le packaging comprend en outre des additifs pertinents pour la nutrition, de façon préférable des vitamines, des substances minérales, des oligo-éléments, des fibres alimentaires, des enzymes, des extraits de plantes, des protéines, des carbohydrates et/ou des graisses.
